# EUROPÄISCHE PATENTSCHRIFT

(11) **EP 0 610 698 B1**
(45) Veröffentlichungstag und Bekanntmachung des Hinweises auf die Patenterteilung: **16.05.2001**
(21) Anmeldenummer: 94100846.8
(22) Anmeldetag: 21.01.1994
(51) Int. Cl.: C07D 471/04, C07D 235/16, A61K 31/415

(54) **Substituierte Imidazo(4,5-b)pyridine und Benzimidazole als Angiotensin II Antagoniste**
Substituted imidazo(4,5-b)pyridines and benzimidazoles as angiotensin II antagonistes
Imidazo(4,5-b)pyridines et benzinimidazoles substitués comme angiotensin II antagonistes

(30) Priorität: 03.02.1993 DE 4302956
(43) Veröffentlichungstag der Anmeldung: 17.08.1994
(73) Patentinhaber: BAYER AG, 51368 Leverkusen (DE)
(72) Erfinder: Müller-Gliemann, Matthias, Dr., D-42697 Solingen (DE); Dressel, Jürgen, Dr., D-42477 Radevormwald (DE); Fey, Peter, Dr., D-42111 Wuppertal (DE); Hanko, Rudolf H., Dr., D-40237 Düsseldorf (DE); Hübsch, Walter, Dr., D-42113 Wuppertal (DE); Krämer, Thomas, Dr., D-42111 Wuppertal (DE); Müller, Ulrich E., Dr., D-42111 Wuppertal (DE); Beuck, Martin, Dr., D-40699 Erkrath (DE); Kazda, Stanislav, Prof. Dr., D-42115 Wuppertal (DE); Wohlfeil, Stefan, Dr., D-40724 Hilden (DE); Knorr, Andreas, Dr., D-40699 Erkrath (DE); Stasch, Johannes-Peter, Dr., D-42651 Solingen (DE); Zaiss, Siegfried, Dr., D-42113 Wuppertal (DE)

(56) Entgegenhaltungen:
- EP-A- 0 253 310
- EP-A- 0 399 732
- EP-A- 0 400 974
- EP-A- 0 513 533
- WO-A-91/11999
- WO-A-91/12002
- US-A- 5 240 938

## Beschreibung

Die Erfindung betrifft substituierte Imidazo[4,5-b]pyridine und Benzimidazole, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die WO 91/11999, WO 91/12002 und die ebenfalls auf die gleiche Anmeldung - wie die beiden zuvor genannten WO-Schriften - zurückgehende US-5,240,938 beschreiben Angiotensin-II-Antagonisten, bei welchen Phenylderivate über eine
Methylengruppe mit einem substituierten Heterocyclus verbunden sind.

Aus der EP-A2-253 310 ist bekannt, daß bestimmte substituierte Imidazolderivate, vor allem durch Chlor substituierte Imidazolderivate, als Angiotensin-II-Blocker wirken und zur Behandlung von Bluthochdruck und kongestiver Herzinsuffienz geeignet sind.

Andere Azolderivate, die als Antagonisten von Angiotensin-II-Rezeptoren fungieren, sind in der EP-A1-485 929 offenbart.

Die EP-A1-399 732 beschreibt unter anderem N-Benzyl substituierte Benzimidazolderivate, welche blutdrucksenkende Eigenschaften besitzen und gegen kongestive Herzinsuffizienz wirken.

Ebenfalls Bluthochdruck senkende Mittel gegen kongestive Herzinsuffizienz und erhöhten Augeninnendruck offenbart die EP-A-400 974. Die dort beschriebenen Wirkstoffe leiten sich von imidazofusionierten sechsgliedrigen Heterocyclen ab.

In der EP-A2-513 533 sind heterocyclisch substituierte Phenylessigsäurederivate, sowie ein Verfahren zu deren Herstellung und deren Verwendung in Arzneimitteln gegen Bluthochdruck und Artheriosklerose offenbart.

Die vorliegende Erfindung betrifft folgende substituierte Imidazo[4,5-b]pyridine und Benzimidazole 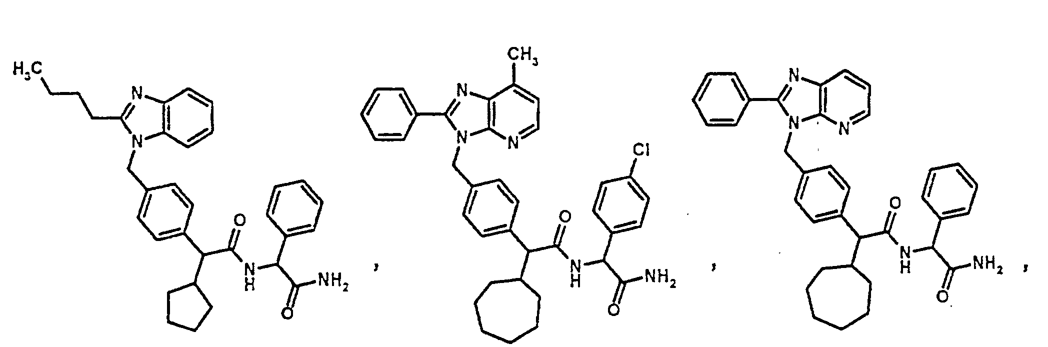 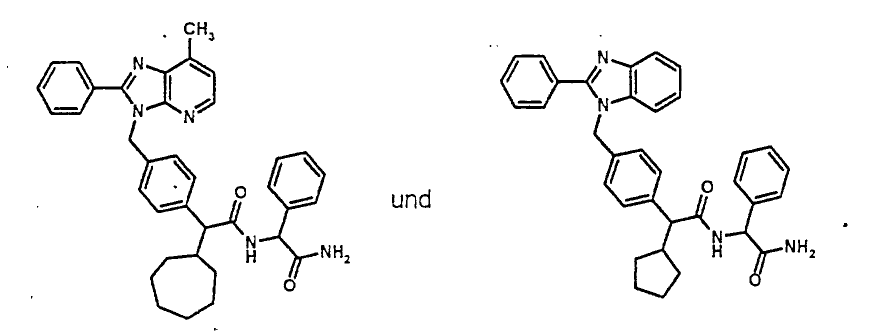 gegebenenfalls in einer isomeren Form und deren Salze.

Die erfindungsgemäßen Verbindungen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der substituierten Imidazo[4,5-b]pyridine und Benzimidazole können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Außerdem wurde ein Verfahren zur Herstellung der erfindungsgemäßen Verbindungen gefunden, dadurch gekennzeichnet, daß man

Verbindungen der Formel (II) 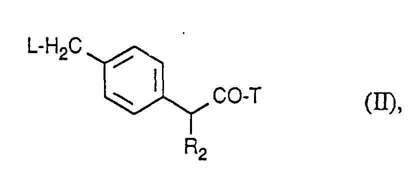 in welcher
- R²: Cyclopentyl oder Cycloheptyl ist,
- L: für eine typische Abgangsgruppe wie beispielsweise Chlor, Brom, Jod, Tosylat oder Mesylat, vorzugsweise für Brom, steht
und
- T: für geradkettiges oder verzweigtes (C₁-C₄)-Alkoxy steht,
zunächst mit Verbindungen der Formel (III) 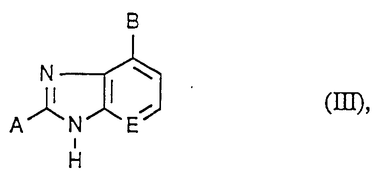 in welcher
A gleich Phenyl oder n-Butyl, B gleich Waserstoff oder Methyl und
E gleich Stickstoff oder CH,
in inerten Lösemitteln, gegebenenfalls in Anwesenheit einer Base und gegebenenfalls unter Schutzgasatmosphäre zu Verbindungen der Formel (IV) 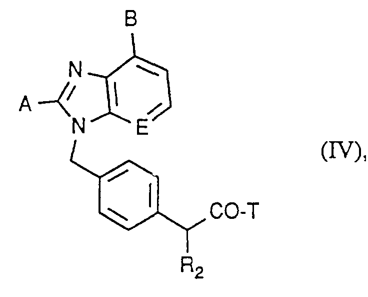 in welcher
A, B, E, T und R² die oben angegebene Bedeutung haben, umsetzt,
und im Fall der Amide ausgehend von den durch Verseifung erhaltenen Säuren, gegebenenfalls unter vorgeschalteter Aktivierung, in inerten Lösemitteln, gegebenenfalls in Anwesenheit einer Base und/oder Dehydratisierungsmittel mit

NH₂-CH(Phenyl)-CONH₂ oder NH₂-CH(p-Cl-Phenyl)-CONH₂,

umsetzt,
und gegebenenfalls die Substituenten A und B nach üblichen Methoden variiert,
und gegebenenfalls die Isomeren trennt, und im Fall der Herstellung der Salze mit einer entsprechenden Base oder Säure umsetzt,
und im Fall der Ester, ausgehend von den aktivierten Carbonsäuren, mit den entsprechenden Alkoholaten umsetzt.

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo-[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat.

Im allgemeinen setzt man die Base in einer Menge von 0,05 mol bis 10 mol, bevorzugt von 1 mol bis 2 mol bezogen auf 1 mol der Verbindung der Formel (III) ein.

Das Verfahren wird im allgemeinen in einem Temperaturbereich von -30°C bis +100°C, bevorzugt von -10°C bis +60°C, durchgeführt.

Das Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumhydroxid eingesetzt.

Als Lösemittel eignen sich für die Verseifung Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Bevorzugt erfolgt die Hydrolyse mit Säuren wie beispielsweise Trifluoressigsäure, Essigsäure, Chlorwasserstoffsäure, Chlorwasserstoffsäure / Dioxan, Bromwasserstoffsäure, Methansulfonsäure, Schwefelsäure oder Perchlorsäure, besonders bevorzugt mit Trifluoressigsäure oder Chlorwasserstoffsäure / Dioxan.

Die Hydrolyse wird im allgemeinen in einem Temperaturbereich von 0°C bis +100°C, bevorzugt von +20°C bis +80°C, durchgeführt.

Im allgemeinen wird die Hydrolyse bei Normaldruck durchgeführt. Es ist aber auch möglich, bei Unterdruck oder bei Überdruck zu arbeiten (z.B. von *0,5* bis 5 bar).

Bei der Durchführung der Verseilung wird die Base im allgemeinen in einer Menge von 1 bis 3 mol, bevorzugt von 1 bis 1,5 mol, bezogen auf 1 mol des Esters, eingesetzt. Besonders bevorzugt verwendet man equimolare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Amidierung und erfolgt im allgemeinen in einem der oben aufgeführten Lösemittel, vorzugsweise in Tetrahydrofuran oder Dichlormethan.

Die Amidierung kann ausgehend von den Verbindungen der Formel (IV) gegebenenfalls über die aktivierte Stufe der Säurehalogenide oder gemischter Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können, verlaufen.

Die Amidierung oder Sulfoamidierung erfolgt im allgemeinen in einem Temperaturbereich von -50°C bis +80°C, vorzugsweise von -30°C bis +20°C, und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der Verbindungen

NH₂-CH(Phenyl)-CONH₂ oder NH₂-CH(p-Cl-Phenyl)-CONH₂

eingesetzt.

Als säurebindende Mittel für die Amidierung oder Sulfoamidierung können Alkalioder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[4.3.0]-nonene-5 (DBN) oder 1,5-Diazabicyclo[3.4.0]undecene-5 (DBU) eingesetzt werden. Bevorzugt ist Triethylamin.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesterazid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der Formel (II) sind teilweise bekannt oder neu und können dann beispielsweise beispielsweise hergestellt werden, indem man die entsprechenden 4-Methylverbindungen im Sinne einer Substitution, beispielsweise durch Halogenierung in Anwesenheit einer der oben aufgeführten Basen und/oder Hilfstoffe und eines der Lösemittel umsetzt

Die Verbindungen der Formeln (III), sowie NH₂-CH(Phenyl)-CONH₂ oder NH₂-CH(p-CI-Phenyl)-CONH₂ sind größtenteils bekannt oder können nach üblichen Methoden hergestellt werden.

Die Verbindungen der Formel (IV) sind neu und können beispielsweise, wie oben beschrieben, hergestellt werden.

Die erfindungsgemäßen Verbindungen zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretions-stimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

| Agonisten und ihre Standardkonzentrationen (Applikationsvolumen pro Einzelgabe = 100 µl): | | |
|---|---|---|
| KCl | 22,7;32,7;42,7;52,7 | mmol/l |
| 1 Noradrenalin | 3x10⁻⁹x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.

Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2), 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

**Tabelle B:**

| Bsp.-Nr. | IC₅₀ [nM] |
|---|---|
| 9 | 43 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösernittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Laufmittel

(A) Dichlormethan / Methanol = 20:1
(B) Dichlormethan / Methanol = 9:1
(C) Dichlormethan / Methanol Eisessig = 9:1:0,1
(D) Dichlormethan / Methanol = 95:5
(E) Petrolether / Essigester = 3:7
(F) Dichlormethan/Methanol/Ammoniak = 9:1:0,1
(G) Essigester
(H) Essigester / Methanol = 10:1
(I) Dichlormethan / Methanol = 10:1
(J) Petrolether / Essigester = 1:1

### Ausgangsverbindungen

### Beispiel I

p-Tolylessigsäuremethylester

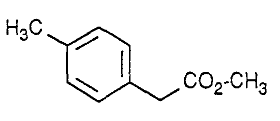

33 g (220 mmol) p-Tolylessigsäure werden in 260 ml 2,2-Dimethoxypropan bei Raumtemperatur gelöst, mit 20 ml konz. HCl versetzt und 2,5 h gerührt. Zur Aufarbeitung wird eingeengt, in Methanol aufgenommen, erneut eingeengt, in Essigester wieder gelöst, über Natriumsulfat getrocknet, filtriert und das Lösemittel entfernt.
Ausbeute: 35,6 g (217 mmol) 99% d.Th.
R_{f} = 0,23 (Toluol / Essigester = 3:1)

### Beispiel II

2-Isopropyl-2-p-tolylessigsäuremethylester

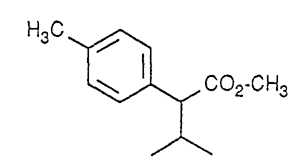

36,2 g (0,20 mol) p-Tolylessigsäuremethylester werden in 150 ml DMF gelöst und bei 0°C unter Rühren zu einer Lösung von 29,6 g (0,26 mol) Kalium-tert.butylat zugetropft. Nach 30 Minuten werden 25 ml (0,26 mol) 2-Brompropan, gelöst in 100 ml DMF langsam zugegeben, sodaß die Temperatur nicht über 5°C steigt. Nach Rühren über Nacht wird zur Aufarbeitung eingeengt, mit Ether und Wasser versetzt, geschüttelt, die organische Phase abgetrennt, die wäßrige Phase noch zweimal extrahiert, und die vereinigten organischen Phasen werden über Natriumsulfat getrocknet, filtiert und eingeengt.
Ausbeute: 36,3 g (0,18 mol) 88% d.Th.
R_{f} = 0,85 (Toluol / Essigester 9:1)

### Beispiel III

2-Isopropyl-2-p-(brommethyl)-phenyl-essigsäure-methylester

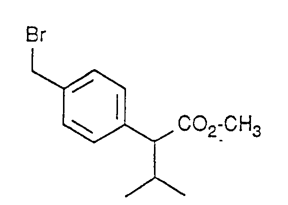

36,2 g (0,18 mol) der Verbindung aus Beispiel II werden in 100 ml Tetrachlorkohlenstoff unter Rückfluß mit 0,9 g (36 mmol) N-Bromsuccinimid und 1,5 g (10 mmol) Azabisisobutyronitril versetzt. Nach Abklingen der Reaktion werden entsprechend noch fünfmal 0,9 g N-Bromsuccinimid (gesamt 0,21 mol) zugegeben. Nach 1 h Reflux wird zur Aufarbeitung auf 0°C gekühlt, filtriert, eingeengt und über Kieselgel 60 (Laufmittel: Petrolether / Ether = 9:1) chromatographiert.
Ausbeute: 37,5 g (0,13 mol) 73% d.Th.
R_{f} = 0,51 (Petrolether / Ether = 9:1)

### Herstellungsbeispiele

### (Nicht erfindungsgemäße Beispiele 1 bis 5 und erfindungsgemäße Beispiele 6 bis 10)

### Beispiel 1

2-[4-{2-Cyclopropyl-5,7-dimethyl-imidazo-[4,5-b]pyridin-3-yl-methyl}phenyl]-2-isopropylessigsäuremethylester 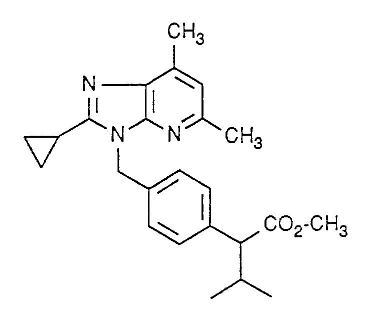 750 mg (4 mmol) 2-Cyclopropyl-5,7-dimethyl-imidazo[4,5-b]pyridin werden in 4 ml DMF gelöst und bei 0°C unter Rühren zu einer Suspension von 120 mg (80%ig, 4 mmol) Natriumhydrid in 28 ml DMF gegeben. Nach 15 Minuten wird eine Lösung von 1,37 g (4,8 mmol) der Verbindung aus Beispiel III in 25 ml DMF zugegeben und noch 2 Stunden bei Raumtemperatur gerührt. Zur Aufarbeitung wird auf Eiswasser gegeben, zweimal mit Essigester extrahiert, die vereinigten organischen Phasen werden fünfmal mit gesättigter wäßriger Kochsalzlösung gewaschen, über Natriumsulfat getrocknet, filtriert, eingeengt und über Kieselgel 60 (Dichlormethan / Methanol = 100:5) chromatographiert.
Ausbeute: 1,03 g (2,6 mmol) 66% d.Th.
R_{f} = 0,91 (Dichlormethan / Methanol = 95:5)

### Beispiel 2

2-[4-{2-Cyclopropyl-5,7-dimethyl-imidazo[4,5-b] pyridin-3-yl-methyl}phenyl]-2-isopropylessigsäure 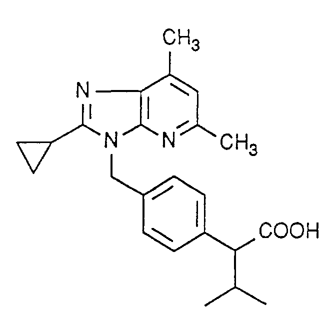 0,9 g (2,3 mmol) der Verbindung aus Beispiel 1 werden in 12 ml Dioxan bei Raumtemperatur mit einer Lösung von 0,19 g (4,6 mmol) Lithiumhydroxid-hydrat in 10 ml Wasser versetzt und über Nacht bei Raumtemperatur gerührt. Zur Aufarbeitung wird mit Ether und Wasser versetzt, geschüttelt, die organische Phase getrennt und noch zweimal mit Wasser extrahiert. Die vereinigten organischen Phasen werden mit 1 N Kaliumhydrogensulfatlösung auf pH 3 eingestellt und dreimal mit Essigester extrahiert. Die vereinigten organischen Phasen werden über Natriumsulfat getrocknet, filtriert und eingeengt
Ausbeute: 0,84 g (2,2 mmol) 97% d.Th.
R_{f} = 0,54 (Dichlormethan / Methanol =9:1)

### Beispiel 3

2-[4-{2-Cyclopropyl-5,7-dimethyl-imidazo[4,5-b]pyridin-3-yl-methyl}phenyl]-2-isopropylessigsäure-p-tolylsulfonamid 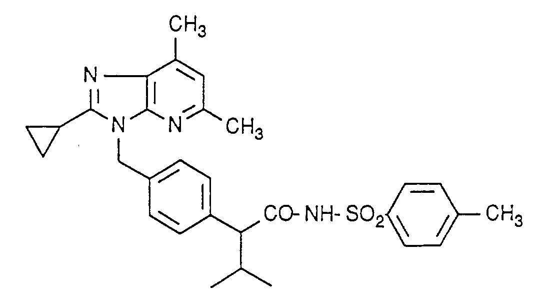 0,41 g (1,1 mmol) der Verbindung aus Beispiel 2 werden unter Argon in einem Gemisch aus 20 ml Dichlormethan und 2,5 ml Tetrahydrofuran gelöst und auf 0°C gekühlt. Man gibt 0,3 ml (2,2 mmol) Triethylamin sowei 0,34 g (1,6 mmol) Dicyclohexylcarbodiimid hinzu und läßt 30 min bei -10°C rühren. Nach Zusatz von 0,22 g (1,3 mmol) p-Toluolsulfonamid, gelöst in 2 ml Dichlormethan, sowie 0,16 g (1,3 mmol) N,N-4-Dimethylaminopyridin, gelöst in 3 ml Dichlormethan, läßt man über Nacht auf Raumtemperatur kommen. Zur Aufarbeitung wird mit Wasser versetzt, geschüttelt, vom Ungelösten abfiltriert und getrennt. Die wäßrige Phase wird noch zweimal mit Dichlormethan extrahiert, und die vereinigten organischen Phasen werden über Natriumsulfat getrocknet, filtriert, eingeengt und über Kieselgel 60 (Petrolether / Essigester = 3:7, später reiner Essigester) chromatographiert
Ausbeute: 0,26 g (0,49 mmol) 45% d.Th.

### Beispiel 4 und Beispiel 5

### 2-[4-{2-Cyclopropyl-5,7-dimethyl-imidazo[4,5-b]pyridin-3-yl-methyl}phenyl]-2-isopropylessigsäure-L-phenylglycinolamid

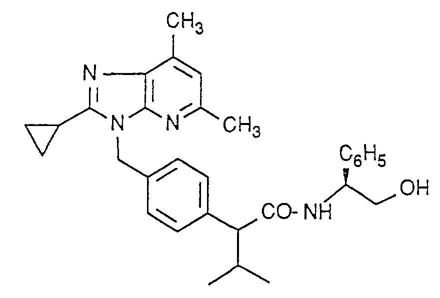

0,41 g (1,1 mmol) der Verbindung aus Beispiel 2 werden unter Argon in einem Gemisch aus 20 ml Dichlormethan und 2,5 ml Tetrahydrofuran gelöst, mit 0,25 g (1,6 mmol) 1-Hydroxy-1H-benzotriazol-monohydrat versetzt und auf 0°C gekühlt. Man gibt 0,3 ml (2,2 mmol) Triethylamin sowie 0,34 g (1,6 mmol) Dicyclohexylcarbodiimid hinzu und läßt 30 min bei -10°C rühren. Nach Zusatz von 0,18 g (1,3 mmol) L-Phenylglycinol in 2,5 ml Dichlormethan läßt man über Nacht auf Raumtemperatur kommen. Zur Aufarbeitung wird mit Wasser versetzt, geschüttelt, vom Ungelösten abfiltriert und getrennt. Die wäßrige Phase wird noch einmal mit Dichlormethan extrahiert, und die verreinigten organischen Phasen werden über Natriumsulfat getrocknet, filtriert und eingeengt und über Kiesegel 60 (Petrolether / Essigester = 7:3, später 3:7) chromatographiert.
Ausbeute: 0,4 g (0,8 mmol) 75% d.Th. Diastereomer A und B
R_{f} = 0,34 (Essigester / Petrolether = 3:7) Diastereomer A (Beispiel 4)
R_{f} = 0,25 (Essigester / Petrolether = 3:7) Diastereomer B (Beispiel 5)

In Analogie zu den nicht erfindungsgemäßen Beispielen 1 bis 5 werden die in Tabelle 1 aufgeführten erfindungsgemäßen Verbindungen 6 bis 10 hergestellt:

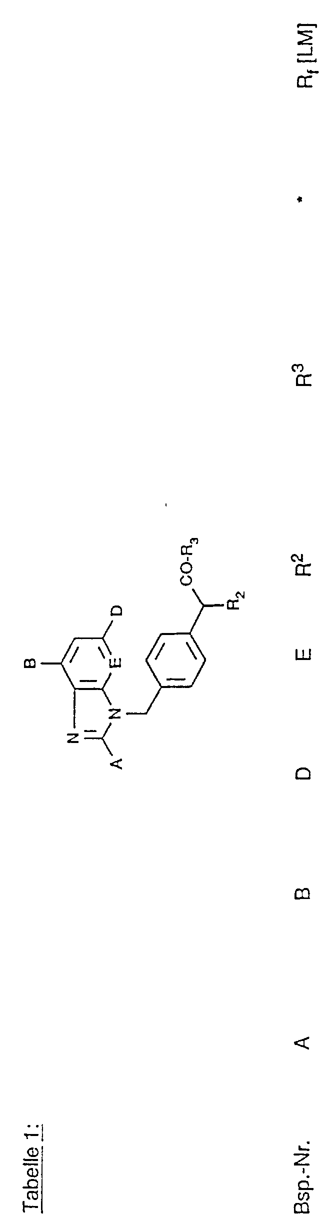

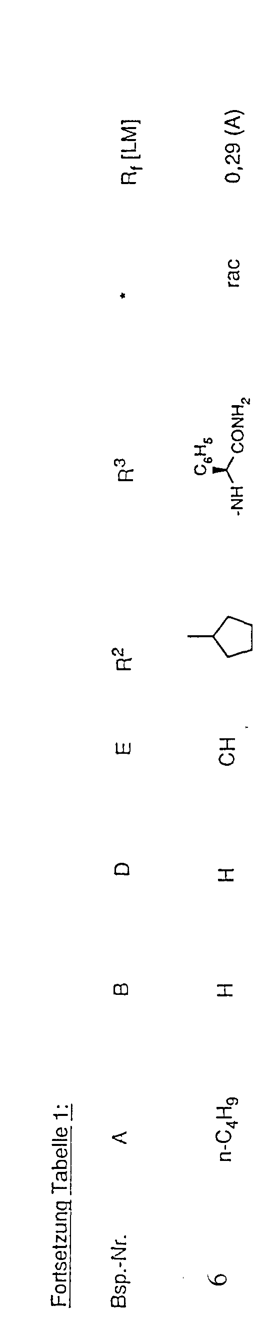

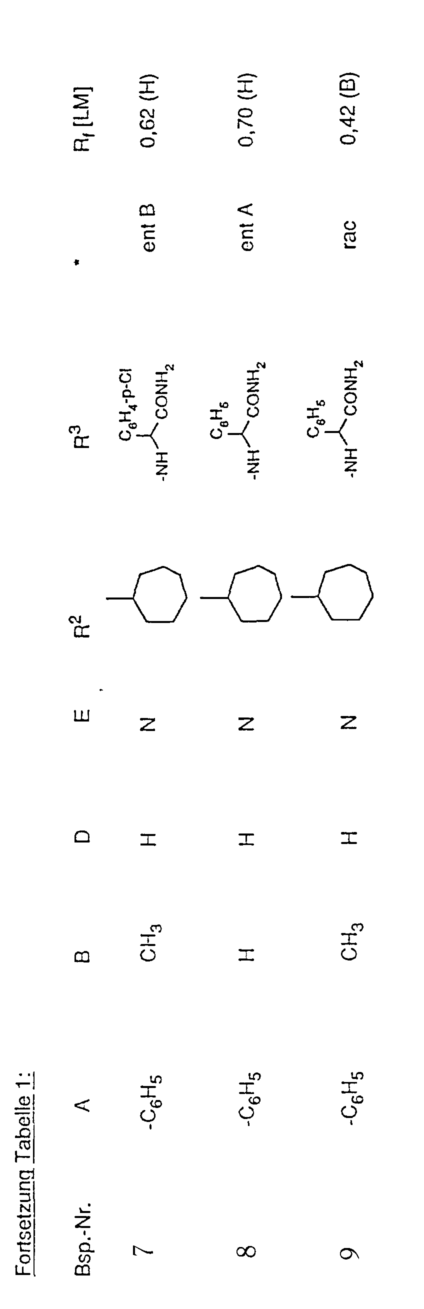

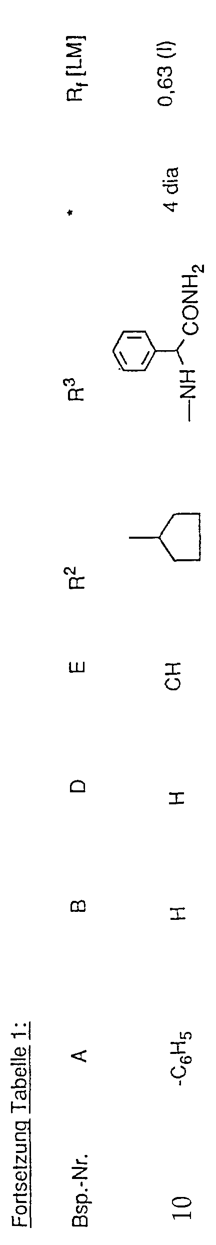

## Patentansprüche

1. Verbindungen der Formeln 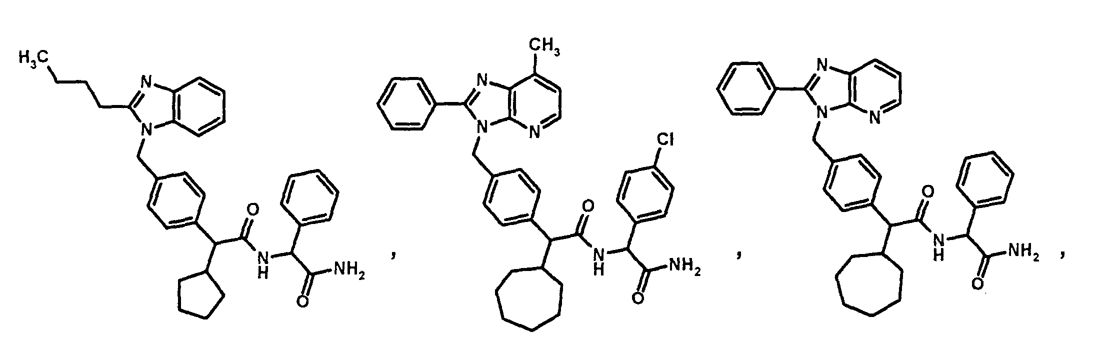 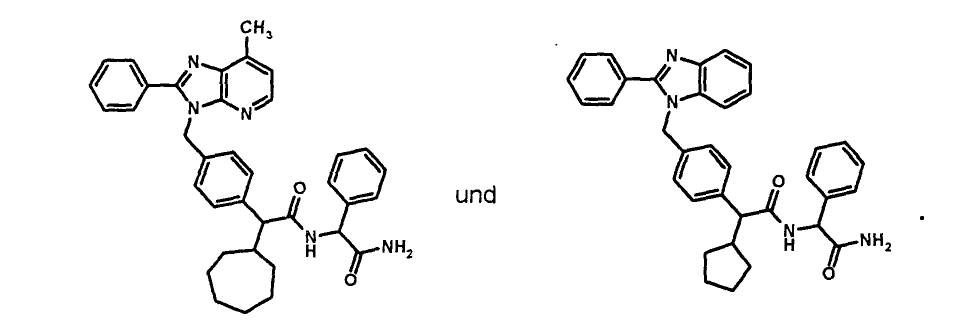 gegebenenfalls in einer isomeren Form und deren Salze.

2. Substituierte lmidazo[4,5]pyridine und Benzimidazole nach Anspruch 1 zur therapeutischen Anwendung.

3. Arzneimittel enthaltend mindestens ein substituiertes lmidazo[4,5-b]pyridin oder Benzimidazol nach Anspruch 1.

4. Arzneimittel nach Anspruch 3 zur Behandlung von arterieller Hypertonie und Atherosklerose.

5. Verwendung von substituierten lmidazo[4,5-b]pyridinen und Benzimidazolen nach Anspruch 1 zur Herstellung von Arzneimitteln.

6. Verwendung nach Anspruch 5 zur Herstellung von Arzneimitteln zur Behandlung von arterieller Hypertonie und Atherosklerose.

7. Arzneimittel nach Anspruch 1 zur Behandlung von ischämischen Gehirnerkrankungen.

8. Verwendung nach Anspruch 5 zur Herstellung von Arzneimitteln zur Behandlung von ischämischen Gehirnerkrankungen.

## Claims

1. Compounds of the formulae 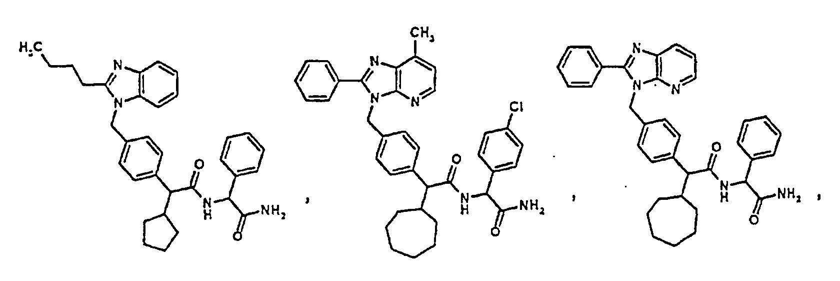 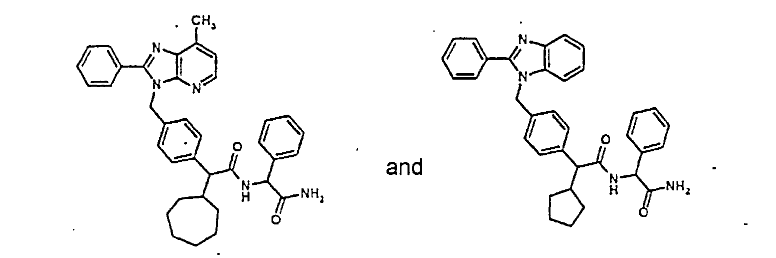 if appropriate in an isometric form and their salts.

2. Substituted imidazo[4,5-b]pyridines and benzimidazoles according to Claim 1 for therapeutic use.

3. Medicaments containing at least one substituted imidazo[4,5-b]pyridine or benzimidazole according to Claim 1.

4. Medicaments according to Claim 3 for the treatment of arterial hypertension and atherosclerosis.

5. Use of substituted imidazo [4,5-b]pyridines and benzimidazoles according to Claim 1 for the production of medicaments.

6. Use according to Claim 5 for the production of medicaments for the treatment of arterial hypertension and atherosclerosis.

7. Medicaments according to Claim 1 for the treatment of ischaemic cerebral disorders.

8. Use according to Claim 5 for the production of medicaments for the treatment of ischaemic cerebral disorders.

## Revendications

1. Composés répondant aux formules 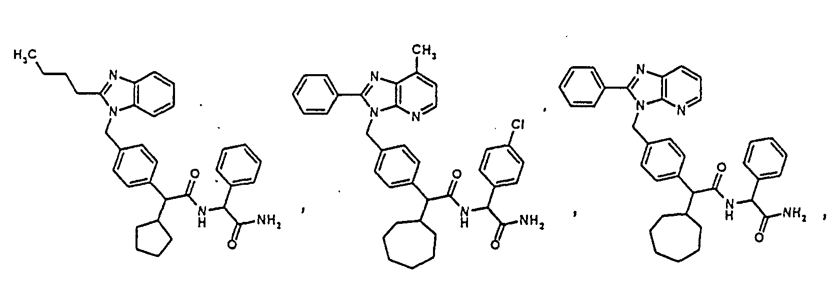 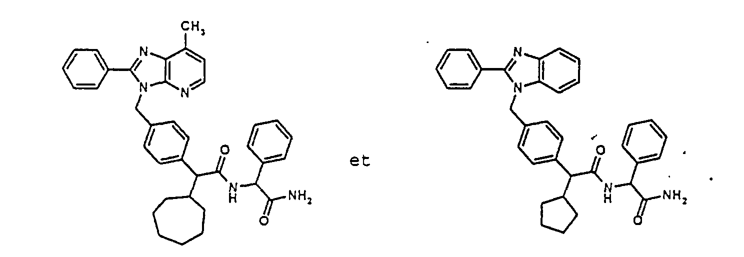 le cas échéant sous une forme isomère, ainsi que leurs sels.

2. Imidazo[4,5]pyridines et benzimidazoles substitués selon la revendication 1, pour l'utilisation thérapeutique.

3. Médicament contenant au moins une imidazo[4,5-b]pyridine ou un benzimidazole substitué selon la revendication 1.

4. Médicament selon la revendication 3 pour le traitement de l'hypertension artérielle et de l'athérosclérose.

5. Utilisation d'imidazo[4,5]pyridines et de benzimidazoles substitués selon la revendication 1, pour la préparation de médicaments.

6. Utilisation selon la revendication 5, pour la préparation de médicaments pour le traitement de l'hypertension artérielle et de l'athérosclérose.

7. Médicament selon la revendication 1 pour le traitement de maladies cérébrales ischémiques.

8. Utilisation selon la revendication 5, pour la préparation de médicaments pour le traitement de maladies cérébrales ischémiques.
